(19) Europäisches Patentamt
European Patent Office
Office européen des brevets

(11) Publication number: **0 198 522**
**A1**

(12) **EUROPEAN PATENT APPLICATION**

(21) Application number: **86200304.3**

(22) Date of filing: **27.02.86**

(51) Int. Cl.⁴: **C 07 D 491/04**, C 07 D 405/04, A 01 N 43/50

(30) Priority: **26.03.85 GB 8507808**

(43) Date of publication of application: **22.10.86 Bulletin 86/43**

(84) Designated Contracting States: **AT BE CH DE FR GB IT LI LU NL SE**

(71) Applicant: **SHELL INTERNATIONALE RESEARCH MAATSCHAPPIJ B.V., Carel van Bylandtlaan 30, NL-2596 HR Den Haag (NL)**

(72) Inventor: **Astles, David Phillip, 37 Longridge, Sittingbourne Kent (GB)**

(74) Representative: **Hunter, Keith Roger Ian et al, 4 York Road, London SE1 7NA (GB)**

(54) **Heterocyclic herbicides.**

(57) Methylenedioxy imidazolinyl acids of the general formula I:

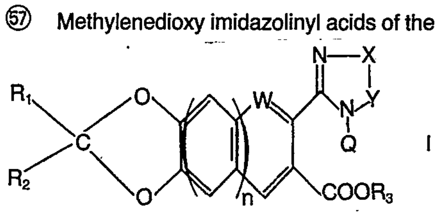

I

wherein W represents CH or N;

n represents zero or 1 when W is N, and represents zero when W is CH;

one of X and Y represents a carbonyl group and the other represents the alkylidene group $=CR_4R_5$;

$R_1$ and $R_2$, which may be the same or different, each independently represents a hydrogen atom or an optionally substituted alkyl or cycloalkyl group;

$R_3$ represents a hydrogen atom or a salt-forming cation or an optionally halogenated alkyl, alkenyl, alkynyl, alkoxyalkyl, cycloalkyl, phenyl, furyl or benzyl group;

$R_4$ represents an alkyl or cycloalkyl group; $R_5$ represents an optionally substituted alkyl, cycloalkyl, alkenyl, phenyl, halophenyl or benzyl group, or $R_4$, $R_5$, and the interjacent carbon atom, together represent a cycloalkyl group; and Q represents a hydrogen atom or an acyl group. Also, processes for the preparation of such compounds, and their use as herbicides.

EP 0 198 522 A1

K 1987 FF

# HETEROCYCLIC HERBICIDES

This invention relates to certain imidazolinyl benzoic acids, the preparation of such compounds, herbicidal compositions containing them, and to their use in controlling undesired plant growth.

UK Patent Application No. 2004537 and European Patent Application 41623 describes imidazolinyl benzoic and nicotinic acids, and their use as herbicides. It has now been found that useful herbicidal activity is present in certain methylenedioxy derivatives of such compounds.

Accordingly, the present invention provides methylenedioxy imidazolinyl acids of the general formula I:-

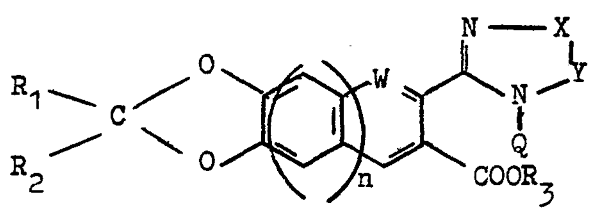

I

wherein W represents CH or N;

n represents zero or 1 when W is N, and represents zero when W is CH;

one of X and Y represents a carbonyl group and the other represents the alkylidene group $=CR_4R_5$;

$R_1$ and $R_2$, which may be the same or different, each independently represents a hydrogen atom, or an optionally substituted alkyl or cycloalkyl group;

$R_3$ represents a hydrogen atom or a salt-forming cation, or an optionally halogenated alkyl, alkenyl, alkynyl, alkoxyalkyl, cycloalkyl, phenyl, furyl, or benzyl group;

$R_4$ represents an alkyl or cycloalkyl group; $R_5$ represents an optionally substituted alkyl, cycloalkyl, alkenyl, phenyl, halophenyl or benzyl group; or $R_4$, $R_5$ and the interjacent carbon atom, together represent a cycloalkyl group; and

Q represents a hydrogen atom or an acyl group.

The term "acyl" is used to denote the radical derived from an organic acid by the removal of a hydroxyl group; the organic acid may be a carboxylic acid (including carbamic acid derivatives) or a sulphonic acid, examples of suitable acyl groups being groups of the formula $-COR_6$ or $-SO_2R_6$ wherein $R_6$ represents a hydrogen atom or an alkyl, haloalkyl, cycloalkyl, alkoxyalkyl, alkoxy, alkylthio, alkylamino, or phenylamino group, or a phenyl or furyl group optionally substituted by a halogen atom or an alkyl, haloalkyl or nitro group.

When any of the above substituents contains an alkyl, alkenyl or alkynyl substituent group, this may be linear or branched and may contain up to 10, preferably up to 6, carbon atoms, suitable examples being methyl, ethyl and propyl, and in the case of an alkyl group may also be substituted by a halogen, especially chlorine, atom or an alkoxy group such as a methoxy group. When they contain a cycloalkyl substituent group this may contain from 3 to 10, preferably 3 to 8, carbon atoms, and is suitably cyclopropyl. When any of the foregoing substituents are designated as being optionally substituted, the substituent groups which are optionally present may be any of those customarily employed in the development of pesticidal compounds, and/or the modification of such compounds to influence their structure/activity, persistence, penetration or other property. Specific examples of such substituents include halogen, especially fluorine, chlorine or bromine, atoms, and nitro, cyano, hydroxyl, alkyl, haloalkyl, and alkoxy groups; in the

case of halogen substituted alkyl groups, a particular preferred example is trifluoromethyl.

When $R_3$ represents a salt-forming cation, this may be derived from any suitable base; convenient cation species including alkali metal atoms and ammonium or alkylammonium.

Preferred compounds are those wherein $R_1$ and $R_2$ each represents a hydrogen atom; n represents 1 when W is N; X represents the alkylidene group $=CR_4R_5$ in which $R_4$ and $R_5$ each independently represents an alkyl group of 1-6 carbon atoms, in particular in which $R_4$ is methyl and $R_5$ is isopropyl; Y represents a CO group; Q represents a hydrogen atom or an acyl group of formula $COR_6$ in which $R_6$ represents an alkyl group of 1-6 carbon atoms, especially a methyl group; and $R_3$ represents a hydrogen atom, an alkyl group of 1-6 carbon atoms, especially methyl, an alkali metal cation such as sodium, or an alkylammonium cation in which the alkyl group or groups contain(s) 1-6 carbon atoms.

The invention also provides processes for the preparation of methylenedioxy imidazolinyl acids as defined above; the precise sequence of process steps depends upon whether W is N or CH. For those compounds wherein W is CH, the process comprises reacting a phthalic anhydride derivative of formula II

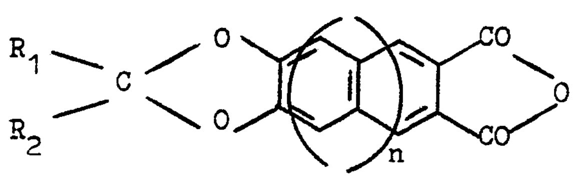

II

with a 2-amino amide of formula III

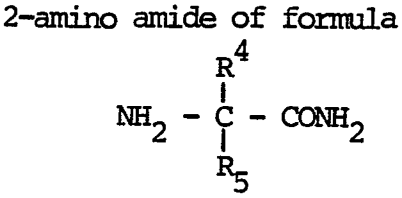

wherein the substituents are as defined for formula I above, and cyclising the intermediate product by treatment with a base. This reaction is conveniently carried out in organic solvent,

preferably polar, such as acetonitrile, and the subsequent cyclisation to the desired imidazole is suitably effected with an alkali metal, preferably sodium, hydroxide.

For those compounds wherein W is N, the process comprises reacting a benzoxazole of formula IV

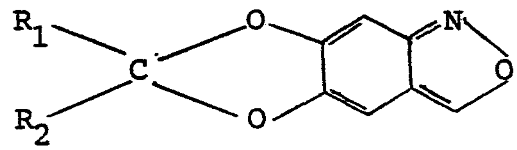

IV

with a maleimide of formula V:

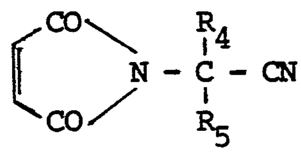

V

followed by hydrolysis in the presence of a strong acid and (cyclo)dehydration. The hydrolysis is conveniently effected by a strong mineral acid, such as concentrated sulphuric acid, and the (cyclo)dehydration is suitably effected by contact with a hydride, such as an alkali metal hydride, in an organic solvent, such as xylene.

Those compounds of formula I wherein Q represents a hydrogen atom can, of course, exist in tautomeric forms, which correspond to the alternatives given for X and Y, and as is clear from the definitions of X and Y, both tautomers fall with the scope of this invention. When Q is an acyl group, the alternative configurations (represented by the alternatives for X and Y) represent different isomers, which again fall within the scope of this invention. Further, when the moieties $R_4$ and $R_5$ differ from each other the compounds of formula I could be resolved into district enantiomers which also fall within the scope of the invention. As is well known, it is frequently the case that one such isomer will have greater biological activity than the other isomer or isomers.

The compound of general formula I have been found to show interesting activity as herbicides. Accordingly, the invention further provides a herbicidal composition comprising a compound of formula I as defined above in association with at least one carrier, and a method of making such a composition which

comprises bringing a compound of formula I into association with at least one carrier.

The invention also provides the use of such a compound or composition according to the invention as a herbicide. Further, in accordance with the invention there is provided a method of combating undesired plant growth at a locus by treating the locus with a compound or composition according to the invention. Application to the locus may be pre-emergence or post-emergence. The dosage of active ingredient used may, for example, be from 0.05 to 4kg/ha. A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example

benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates

and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension conentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope

of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The invention is illustrated in the following Examples.

Example 1

Methyl-2-(5-isopropyl-5-methyl-4-oxo-2-imidazolin-2-yl)-[1,3]-dioxolo[4,5-g] quinoline-3-carboxylate

A) 6-Nitropiperonal (5g) was added over 20 minutes in the dark, with stirring at 10-15°C, to stannous chloride (20g) and concentrated hydrochloric acid (50cc). The reaction mixture was stirred overnight to room temperature, and poured into a mixture of ice-water and chloroform. The chloroform layer was separated, washed, dried and solvent evaporated off to leave a solid which was chromatographically purified to yield, as a solid m.pt. 111-112°C, the desired intermediate: 4,5-methylenedioxy benzoxazole.

B) The benzoxazole product of A (3.7g) was mixed with N-(2-cyano-isopent-2-yl) maleimide (4.36g) in xylene (20cc) and the mixture refluxed under nitrogen for 29 hours. The reaction mixture was cooled, and the precipitate filtered off and washed several times to yield, as a solid m.pt. 240-241°C, the desired intermediate of structure:-

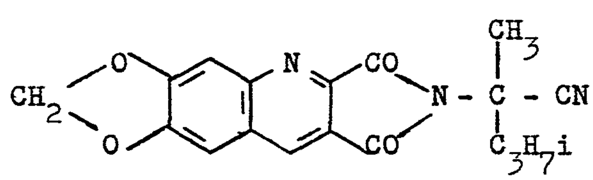

This structure was confirmed by NMR, IR, and $^{13}C$ analysis.

C) The "imide-nitrile" product of B (2.1g) was dissolved in concentrated sulphuric acid (4cc) and stirred at room temperature for 3 hours. The reaction mixture was poured into toluene (30cc)/water (200cc), stirred for 1 hours, and filtered. The solid product was washed to yield, as a solid m.pt. 235-8 (dec.), the desired intermediate

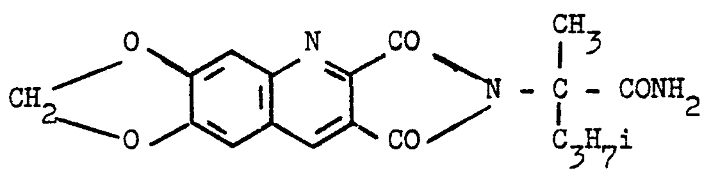

This structure was confirmed by NMR spectra.

D) The "imide-amide" product of C (2.0g)dissolved in xylene (100cc) was heated to reflux in an apparatus fitted with a Dean-Stark take-off head. After 30 minutes at reflux, the reaction mixture was cooled to about 100°C and sodium hydride (0.26g) added with vigorous stirring. After heating for 1 hour under azeotropic conditions a further 0.26g of sodium hydride was added, and reflux continued for 3 hours, followed by a further 0.26g hydride and a further 1 hour at reflux. The xylene was evaporated off, methanol (50ml) added together with a further 0.26g hydride, and the mixture refluxed for 1 hour. The methanol was evaporated off and the residual solid chromatographically purified to yield as a solid m.pt. 202-204°C, the desired final product having the structure:-

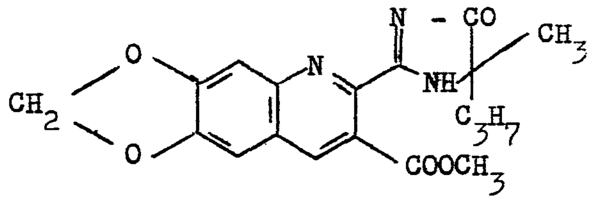

This structure was confirmed by NMR, IR and mass analysis.

Analysis Calc. C 62.0; H 5.2; N 11.4%

Found C 62.0; H 5.6: N 10.5%

Example 2

2-(5-methyl-5-isopropyl-4-oxo-2-imidazolin-2-yl)-[1,3]-dioxolo[4,5-g] quinoline-3-carboxylic acid

Following a procedure similar to that of Example 1, but treating the reaction mixture from stage D) with sodium hydroxide (2M solution) followed by concentrated HCl, yielded the corresponding free acid as a solid, m.pt. 288-290°C.

Analysis Calc. C 60.8; H 4.8; N 11.8%

Found C 60.8; H 4.6; N 11.2%

Example 3

Methyl-2-(1-acetyl-4-isopropyl-4-methyl-5-oxo-2-imidazolin-2-yl)-[1,3]-dioxolo[4,5-g] quinoline-3-carboxylate

The methyl ester product of Example 1 (0.27g) was dissolved in tetrahydrofuran and triethylamine (0.1cc) added followed by, at 0-5°C, a solution of acetyl chloride (0.06g) in benzene (1cc). The reaction mixture was stirred for 15 minutes at 5°C, and then overnight at room temperature. The precipitate was filtered off, and the tetrahydrofuran evaporated off to yield a solid, which was recrystallised to yield the desired acetyl derivative as a solid, m.pt. 190-192°C

Analysis Calc. C 61.3; H 5.1; N 10.2%

Found C 62.1; H 5.7; N 10.1%

This structure was confirmed by NMR.

Example 4

Sodium-2-(5-isopropyl-5-methyl-4-oxo-2-imidazolin-2-yl)-[1,3]-dioxolo[4,5-g] quinoline-3-carboxylic acid (salt)

The free acid product of Example 2 (0.24g), dissolved in water (2cc) was treated at room temperature with a solution of sodium hydroxide (0.027g) in water (2cc) and the reaction mixture stirred overnight. The aqueous layer was recovered, washed and evaporated to dryness, to yield the desired sodium salt as a solid, m.pt. 300-304°C.

Analysis Calc. C 52.3; H 4.8; N 10.1%
Found C 51.5; H 4.2; N 11.1% (2 mole water)

Examples 5 and 6

Following a procedure similar to that described in Example 4, the diisopropyl ammonium (A) and isopropylammonium (B), salts of the Example 2 acid were prepared.

A) M.Pt = >300°C. No analytical results.

B) M.Pt = 296-298°C.

Analysis Calc. C 60.9; H 6.3; N 14.5%
Found C 56.7; H 6.8; N 12.3%

Example 7

2-(5-isopropyl-5-methyl-4-oxo-2-imidazolin-2-yl)-[1,3]-dioxolo [4,5-d]benzoic acid

A) 4,5-dihydroxy phthalic acid, dimethyl ester (1g) in dimethylsulphoxide (7cc) was treated with methylene dichloride (0.4g), then sodium hydroxide (0.18g) and the reaction mixture heated under nitrogen on an oil-bath at 120°C for 90 minutes with stirring. The DMSO solvent was evaporated off at 2mm Hg pressure, and the residue chromatographically purified to yield, as a solid, the desired intermediate 4,5-methylenedioxy phthalic acid, dimethyl ester. This product was then hydrolysed with sodium hydroxide to yield the corresponding free acid as a solid, m.pt. 178.180°C.

B) The 4,5-methylenedioxy phthalic acid obtained in A (1.18g) was treated with acetic anhydride (20cc), and the reaction mixture refluxed for 1 hour 45 minutes. The product was

filtered and the acetic anhydride removed in vacuo, to yield the corresponding phthalic anhydride.

C) 4,5-methylenedioxy phthalic anhydride obtained in B (1.0g) and 2-amino-2,3-dimethyl butyramide (0.73g) were mixed in acetonitrile (5cc), and the reaction mixture stirred at 60°C for 2 hours. The solvent was evaporated, the residue dissolved in 3N sodium hydroxide solution (8cc), and heated at 80-85°C for 3 hours. The reaction mixture was then cooled, filtered, neutralised with concentrated HCl to yield the desired product (free acid) as a solid m.pt. 241-243°C.

| | | | | |
|---|---|---|---|---|
| Analysis | Calc. | C 59.2; | H 5.3; | N 9.2% |
| | Found | C 58.5; | H 4.9; | N 8.5% |

Example 8

Methyl 2-(5-isopropyl-5-methyl-4-oxo-2-imidazolin-2-yl)-[1,3]-dioxolo[4,5-d] benzoate

The free acid product of Example 7 (0.3g), suspended in tetrahydrofuran (20cc) was treated with a solution of diazomethane in ether at 0°C with stirring. The reaction mixture was stirred for 1 hour, then left overnight, and the solvent evaporated. The residue was chromatographically purified to yield the desired methyl ester as a solid, m.pt. 160-162°C.

| | | | | |
|---|---|---|---|---|
| Analysis | Calc. | C 60.4; | H 5.7; | N 8.8% |
| | Found | C 57.2; | H 5.4; | N 8.1% |

Example 9

Herbicidal Activity

To evaluate their herbicidal activity, compounds according to the invention were tested using as representative range of plants: maize, Zea mays (Mz); rice, Oryza sativa (R); barnyard grass, Echinochloa crusgalli (BG); oat, Avena sativa (O); linseed, Linum usitatissisum (L); mustard, Sinapsis alba (M); sugar beet, Beta vulgaris (SB) and soya bean, Glycine max (S).

The tests fall into two categories, pre-emergence and post-emergence. The pre-emergence tests involved spraying a

liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in Table 1.

Table 1

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 1 | 6 | 7 | 7 | 6 | 6 | 8 | 7 | 4 | 5 | 7 | 6 | 7 | 6 | 7 | 7 | 8 | 2 | 7 | 8 | 8 | 7 | 7 | 7 | 6 | 0 |
| | | | | | | | | | 1 | 5 | 4 | 4 | 5 | 6 | 5 | 6 | 0 | 5 | 4 | 5 | 5 | 6 | 4 | 6 | 0 |
| 2 | 7 | 7 | 9 | 7 | 7 | 9 | 8 | 6 | 5 | 7 | 6 | 7 | 7 | 6 | 7 | 7 | 4 | 8 | 9 | 8 | 7 | 9 | 7 | 8 | 8 |
| | | | | | | | | | 1 | 6 | 6 | 7 | 6 | 6 | 7 | 7 | 2 | 8 | 9 | 8 | 7 | 8 | 7 | 8 | 8 |
| 3 | 5 | 5 | 7 | 6 | 6 | 7 | 8 | 0 | 5 | 6 | 5 | 7 | 5 | 6 | 6 | 7 | 3 | 8 | 5 | 7 | 6 | 8 | 4 | 8 | 0 |
| | | | | | | | | | 1 | 4 | 2 | 5 | 3 | 5 | 3 | 5 | 1 | 4 | 2 | 4 | 4 | 5 | 2 | 4 | 0 |
| 4 | 5 | 5 | 8 | 4 | 6 | 8 | 8 | 6 | 5 | 6 | 4 | 8 | 4 | 6 | 8 | 8 | 3 | 8 | 9 | 8 | 6 | 9 | 7 | 8 | 5 |
| | | | | | | | | | 1 | 4 | 4 | 7 | 4 | 6 | 8 | 7 | 2 | 8 | 8 | 8 | 5 | 8 | 7 | 8 | 2 |
| 5 | | | | | | | | | 5 | 7 | 7 | 9 | 7 | 6 | 8 | 9 | 3 | 9 | 9 | 9 | 6 | 8 | 7 | 8 | 6 |
| | | | | | | | | | 1 | 6 | 6 | 7 | 3 | 6 | 7 | 8 | 1 | 8 | 9 | 8 | 5 | 8 | 7 | 8 | 5 |
| 7 | | | | | | | | | 5 | 3 | 3 | 6 | 2 | 4 | 7 | 7 | 4 | 6 | 9 | 7 | 5 | 7 | 7 | 8 | 1 |
| | | | | | | | | | 1 | 2 | 2 | 5 | 1 | 3 | 7 | 6 | 0 | 1 | 8 | 3 | 0 | 3 | 4 | 7 | 0 |

0198522

K 1987 FF

## CLAIMS

1. Methylenedioxy imidazolinyl acids of the general formula I:-

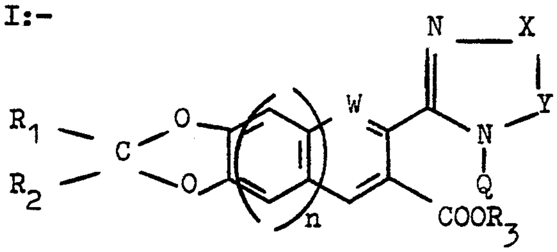

I

wherein W represents CH or N;
n represents zero or 1 when W is N, and represents zero when W is CH;
one of X and Y represents a carbonyl group and the other represents the alkylidene group = $CR_4R_5$;
$R_1$ and $R_2$, which may be the same or different, each independently represents a hydrogen atom or an optionally substituted alkyl or cycloalkyl group;
$R_3$ represents a hydrogen atom or a salt-forming cation, or an optionally halogenated alkyl, alkenyl, alkynyl, alkoxyalkyl, cycloalkyl, phenyl, furyl or benzyl group;
$R_4$ represents an alkyl or cycloalkyl group; $R_5$ represents an optionally substituted alkyl, cycloalkyl, alkenyl, phenyl, halophenyl or benzyl group, or $R_4$, $R_5$, and the interjacent carbon atom, together represent a cycloalkyl group; and Q represents a hydrogen atom or an acyl group.

2. Compounds as claimed in claim 1 wherein $R_1$ and $R_2$ each represents a hydrogen atom; n represents 1 when W is N; and Y represents a carbonyl group.

3. Compounds as claimed in claim 1 or 2 wherein Y represents a CO group and X represents the alkylidene group $= CR_4R_5$ in which $R_4$ and $R_5$ each independently represents an alkyl group of 1-6 carbon atoms; Q represents a hydrogen atom or an acyl group of formula $COR_6$ in which $R_6$ represents an alkyl group of 1-6 carbon atoms; and $R_3$ represents a hydrogen atom, an alkali metal cation, an alkyl group of 1-6 carbon atoms, or an alkylammonium cation.

4. Compounds as claimed in claim 3 wherein $R_4$ represents a methyl group; $R_5$ represents an isopropyl group; and Q represents a hydrogen atom or an acetyl group.

5. Compounds as defined in claim 1 and specifically named in any one of Examples 1 - 8 herein.

6. Process for the preparation of a compound of the general formula I as defined in claim 1, wherein W is CH, which comprises reacting a phthalic anhydride derivative of formula II:-

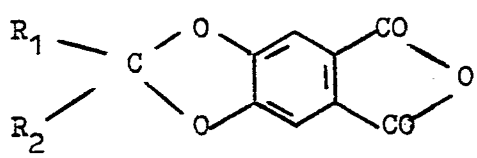

II

with a 2-amino amide of formula III

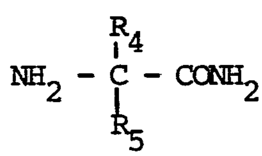

III

the substituents in formulae II and III having the meanings as defined for formula I in claim 1, and cyclising the intermediate product by treatment with a base.

7. Process as claimed in claim 6 wherein the reaction is carried out in a polar organic solvent.

8. Process as claimed in claim 6 or 7 wherein the intermediate product is cyclised by contact with an alkali metal hydroxide

9. Process as defined in claim 6 carried out substantially as hereinbefore described with particular reference to Example 7.

10. Process for the preparation of a compound of the general formula I as defined in Claim 1 wherein W is N, which comprises reacting a benzoxazole of formula IV

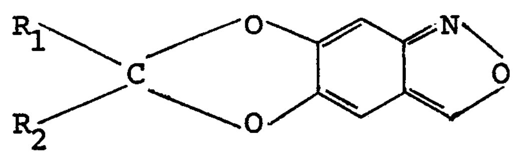

IV

with a maleimide of formula V:

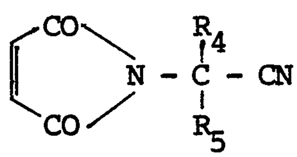

V

followed by hydrolysis in the presence of a strong acid and (cyclo)dehydration.

11. Process as claimed in claim 10 wherein the strong acid is a strong mineral acid.

12. Process as claimed in claim 10 or 11 wherein the (cyclo)dehydration is effected by contact with an alkali metal hydride, in an organic solvent.

13. Process as defined in claim 10 carried out substantially as hereinbefore described with particular reference to Examples 1 - 4.

14. A compound as defined in claim 1, whenever prepared by a process as claimed in any one of claims 6-13.

15. Herbicidal composition, which comprises a compound as claimed in any of claims 1 to 5 or 14, together with a carrier.

16. A composition as claimed in claim 15, which comprises at least two carriers, at least one of which is a surface-active agent.

17. Method of combating undesired plant growth at a locus, which comprises treating the locus with a compound as claimed in any one of claims 1 to 5, or 14, or a composition as claimed in either of claims 15 or 16.

18. The use of a compound as claimed in any one of claims 1 to 5, or 14, as a herbicide.

European Patent Office

# EUROPEAN SEARCH REPORT

0198522

Application number

EP 86 20 0304

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB-A-1 076 828 (WARNER-LAMBERT)<br>--- | | C 07 D 491/04<br>C 07 D 405/04<br>A 01 N 43/50 |
| A | US-A-4 234 589 (HOEHN)<br>--- | | |
| D,A | GB-A-2 004 537 (AMERICAN CYANAMID)<br>----- | | |
| | | | TECHNICAL FIELDS SEARCHED (Int. Cl.4) |
| | | | C 07 D 491/00<br>C 07 D 405/00<br>A 01 N 43/00 |

The present search report has been drawn up for all claims

| Place of search | Date of completion of the search | Examiner |
|---|---|---|
| THE HAGUE | 04-07-1986 | DE BUYSER I.A.F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82